# EUROPEAN PATENT APPLICATION

(11) **EP 4 586 260 A1**
(43) Date of publication of application: **16.07.2025**
(21) Application number: 25150790.1
(22) Date of filing: 08.01.2025
(51) Int. Cl.: G16H 10/20

(54) **SYSTEMS AND METHODS FOR IMPLEMENTING DRUG MECHANISMS OF ACTION WITH MACHINE LEARNING**

(30) Priority: 10.01.2024 US 202463619490 P; 30.05.2024 US 202418678419
(71) Applicant: Icon Clinical Research Limited, Dublin 18 D18 X5R3 (IE)
(72) Inventor: MALOUVIER, Alexandre, Dublin (IE); RAMANNA, Kusuma Manavalli, Dublin (IE); PHILLIPS, Michael, Dublin (IE); QUINN, Gerard, Dublin (IE)
(74) Representative: Weldon O'Brien Ltd.

(57) **Abstract**

A computer-implemented method for generating machine-learning training data includes obtaining (654) mechanism of action (MOA) data that is indicative of a hierarchical tree structure of relationships between the MOA data (138); and generating (656) linear representations of branches of the hierarchical tree structure. It also includes determining (606) association rules for the MOA data by applying one or more frequent pattern-mining algorithm to the linear representations. It also includes determining (308, 608), as at least a portion of the generated machine learning training data, MOA clusters (400) by applying a clustering model (150) to the linear representations and the association rules. The method also includes determining the hierarchical tree structure by extracting (104) a plurality of nodes from the MOA data (138), and generating (602) the hierarchical tree structure based on the extracted nodes.

## Description

### FIELD

The present disclosure relates to implementing drug mechanisms of action with machine learning. More particularly, techniques of the present disclosure relate to methods and systems for generating a machine learning model for predicting whether a post-marketing requirement (PMR) will be imposed on a clinical trial.

### BACKGROUND

As part of the regulatory approval for a pharmaceutical drug, international regulators (e.g., U.S. Food & Drug Administration (FDA) and the European Medicines Agency (EMA)) may have guidelines and processes for a drug to become legal to distribute. An aspect of this process may include determining when and how to impose additional research requirements after marketing approval of a drug. In their guidance for industry, the FDA or EMA may perform a risk assessment during the approval process for a drug which can lead to a request for a post-marketing study to gather more information about the risks of a particular drug. The purpose of such post-marketing requirements (PMRs) may be to better inform product labelling.

Imposition of a PMR may result in significant costs, time and effort. Generally, it is difficult to predict whether a PMR will be required for a particular drug. Conventional techniques for determining whether PMRs may be implemented may be a time-consuming process. For example, an expert may need to evaluate a pharmaceutical asset against the detailed guidelines provided by the FDA or EMA to determine whether a PMR may arise. Such predictions, even if possible, may be inaccurate, subjective, and/or of limited institutional value.

The present disclosure is directed to addressing one or more challenges such as the above. The background description provided herein is for the purpose of generally presenting the context of the disclosure. Unless otherwise indicated herein, the materials described in this section are not prior art to the claims in this application and are not admitted to be prior art, or suggestions of the prior art, by inclusion in this section.

### SUMMARY

We describe in various examples, a computer-implemented method for generating machine learning training data, the method comprising:
obtaining mechanism of action (MOA) data that is indicative of a hierarchical tree structure of relationships between the MOA data;
generating linear representations of branches of the hierarchical tree structure;
determining association rules for the MOA data by applying one or more frequent pattern mining algorithm to the linear representations; and
determining, as at least a portion of the generated machine learning training data, MOA clusters by applying a clustering model to the linear representations and the association rules.

In some examples the method further comprises further comprises:
determining the hierarchical tree structure by:
extracting a plurality of nodes from the MOA data; and
generating the hierarchical tree structure based on the extracted nodes.

In some examples, generating linear representations of branches of the hierarchical tree structure includes applying one or more techniques selected from the group consisting of: tokenization, vectorization, max and min n-gram limit determination, word clouds, median treatments, segmentation, text classification, and categorical transformation.

In some examples, determining association rules for the linear representations comprises applying a Frequent Pattern (FP) Growth algorithm.

In some examples, determining MOA clusters comprises applying a clustering model selected from the group consisting of: a Gaussian Mixture Model (GMM), K-Means Clustering, and hierarchical clustering.

In some examples, the method further comprises transforming the MOA clusters into numerical representations for use as input into a machine learning model.

In some examples, the machine learning training data further includes a labeled dataset indicating whether a post-marketing requirement (PMR) was imposed on a previous clinical trial.

We also describe a computer-implemented method for predicting whether a post-marketing requirement (PMR) will be imposed on a clinical trial, the method comprising:
obtaining data associated with a clinical trial;
obtaining mechanism of action (MOA) data associated with the clinical trial, the MOA data indicative of a hierarchical tree structure of relationships between the MOA data;
generating a linear representation of one or more branches of the hierarchical tree structure; and
generating a prediction of whether a PMR will be imposed on the clinical trial by applying a trained machine learning model to the data associated with the clinical trial and the linear representation of the one or more branches, the trained machine learning model having been trained based on clusters of linear representations of historical MOA data.

We also describe a computer-implemented method as wherein the method includes predicting whether a post-marketing requirement (PMR) will be imposed on a clinical trial, the method comprising:
obtaining data associated with a clinical trial, and said (MOA) data is associated with the clinical trial, and
generating a prediction of whether a PMR will be imposed on the clinical trial by applying a trained machine learning model to the data associated with the clinical trial and the linear representation of the one or more branches, the trained machine learning model having been trained based on clusters of linear representations of historical MOA data according to said generated machine learning training data.

In some examples, the data associated with the clinical trial includes one or more of: global approval status, key regulatory events, therapeutic class, license country, originator country, and target.

In some examples, the trained machine learning model was further trained using regulatory data from one or more of the Food and Drug Administration (FDA) or the European Medicines Agency (EMA).

In some examples, the method further comprises:
causing a user interface of a user device to display the prediction.

In some examples, the trained machine learning model includes a gradient-boosting decision tree model.

In some examples, the prediction further includes an indication of a specific type of PMR likely to be imposed.

We also describe a system for predicting whether a post-marketing requirement (PMR) will be imposed on a clinical trial, the system comprising:
one or more processors; and
a non-transitory computer-readable medium storing instructions that are executable by the one or more processors to perform operations, including:
   obtaining data associated with a clinical trial;
   mechanism of action (MOA) data associated with the clinical trial, the MOA data indicative of a hierarchical tree structure of relationships between the MOA data;
   generating a linear representation of one or more branches of the hierarchical tree structure; and
   generating a prediction of whether a PMR will be imposed on the clinical trial, by applying a trained machine learning model to the data associated with the clinical trial and the linear representation of the one or more branches, the trained machine learning model having been trained based on clusters of linear representations of historical MOA data.

In some examples, the data associated with the clinical trial includes one or more of: global approval status, key regulatory events, therapeutic class, license country, originator country, and target.

In some examples, the trained machine learning model was further trained using regulatory data from one or more of the Food and Drug Administration (FDA) or the European Medicines Agency (EMA).

In some examples, the one or more processors are configured to periodically retrain the trained machine learning model with updated data, wherein the updated data includes one or more of: MOA data, linear representations of MOA data, clusters of linear representations of MOA, data associated with a clinical trial, and regulatory data.

In some examples, the system further comprises:
an interactive user interface configured to receive a drug query from a user and to display a prediction of whether a PMR will be imposed on a clinical trial associated with the drug query.

In some examples, the interactive user interface is further configured to display an indication of a specific type of PMR likely to be imposed.

In some examples, the interactive user interface is further configured to display details of historical post-marketing studies and types of studies mandated for drugs associated with the drug query.

In some examples, the system is configured to receive data from one or more data sources such as sources of data of completed, in progress, and/or planned clinical trials. The system may further be configured to perform data extraction through a data extraction model from the one or more data sources. The data extraction model may include web scrapers, zip file processors, an application programming interface (API) connector, a JavaScript Object Notation (JSON)/Extensible Markup Language (XML) parser.

In some examples, the extracted data is sent to a data transformation model which processes the data prior to inputting the data in the machine learning (ML) predictive model.

For some embodiments, the system includes an ML predictive model which receives processed data from a transformed data repository, and the ML predictive model implements techniques such as gradient-boosting decision tree methodology, incorporating training data such as the data engineered in the other processing steps to identify factors associated with the occurrence of PMRs. Once trained, the ML predictive model may provide as output, a PMR prediction for any drug query made by an end user of the system. Each prediction may be expressed as a probability score, which may be determined by the ML predictive model. The PMR prediction may then, for example, be output to a user interface.

In some embodiments, the user interface enables one or more users to access the system 100 to perform searches of a pharmaceutical drug, to allow a user to perform a search of the system based on an originator license, company, drug, drug family, and/or drug MOA.

The extracted data may be staged in a staging database, from where the data is joined using relevant keys, transformed and saved into a transformed data repository for consumption by the ML model.

The system may, for example, perform scheduled data capture (e.g., from the one or more data sources) at set time intervals, and/or it may be automatic, e.g., performed in response to detection of new data being available; and/or in response to a user instruction.

In some embodiments, real world data includes data extracted from a workflow and it may include history data extracted from a data workflow. A feature engineering model may, for example, be applied by a data transformation model, and the output of the feature engineering model, along with history data may be fed to the predictive model for analysis.

In certain embodiments, the feature engineering model receives real world data for processing. This may include some or all historical drug MOAs, their respective logical relationships, as well as MOAs related to a particular new pharmaceutical drug. The particular drug MOA relationships may include one or more parent/child relationships. Received drug MOAs may have associations that can be modeled using feature engineering. The feature engineering model may, for example, model the MOA data as drug MOA hierarchies. The MOA hierarchical tree structure may depict a multi-level tree structure with interconnected synonym nodes of MOAs.

In some examples, a first Natural Language Processing (NLP) model is configured to reduce the MOA hierarchies to linear representations. The first NLP model may utilize one or more exemplary algorithms to reduce the MOA hierarchical tree structures to linear representations. For example, in certain embodiments, the first NLP model utilize algorithms including, but not limited to: tokenization, vectorization, max and min n-gram limit determinations, word clouds, median treatment, segmentation, text classification, and/or categorical transformation. In some embodiments, the linear representations are then be fed into a frequent pattern mining algorithm, e.g., by the Frequent Pattern (FP) Growth algorithm, in order to extract association rules. As used herein, association rules generally encompass identified relationships between items in a given dataset representing patterns where items in a dataset frequently appear together, indicating that the presence of certain items (antecedents) likely predicts the presence of others (consequents). In some embodiments, association rules may be evaluated, e.g., based on a high confidence such as a factor greater than 50%, and where a consequent is set to pre-identified PMRs.

The outputs of the first NLP model, e.g., the linear representations and the association rule data, may, for example, be saved and/or fed into a machine learning clustering model. In some embodiments, the machine learning clustering model utilizes machine learning algorithms such as clustering to categorize the MOAs into a set of distinct mechanism groups, e.g., based on silhouette scores. In certain embodiments, raw text fields are transformed into a document-term matrix, e.g., based on a scikit-learn TfidfVectorizer. In some embodiments, the data are reduced to a binary representation in which frequency of the n-grams is not the deterministic factor. In alternate embodiments, a word analyzer is be utilized, e.g., instead of a character analyzer, to speed up the process. The MOA clusters clustered groups may then be output, e.g., in a numerical representation, to the ML predictive model 112 for further analysis. An experimental result of the foregoing procedure resulted in clusters having strong relationships with therapeutic indicators.

In some embodiments, the feature engineering model also has a second NLP model, which may, for example, receive RWD of drug regulatory events, drug therapeutic classes, drug targets, and drug licensing and origin from real world data. The second NLP model may be configured to embed text variables as numeric vectors. For example, the second NLP model may apply techniques including, but not limited to: hot encoding, text processing, n-Gram optimization, median treatments, segmentation, numerical-categorical transformation. The outputs of certain embodiments of the second NLP model are fed to the ML predictive model, e.g., in combination with the other data discussed above, such as the MOA data, numerical representation of clusters.

The ML predictive model may be configured to utilize a gradient-boosting decision tree methodology to use the training data generated in the other processing steps to identify factors associated with the occurrence of PMRs. Once trained, the ML predictive model may provide a PMR prediction for any drug query made by an end user of the system, as outputted in the user interface. Each prediction may be expressed as a probability score, which may be determined by the model's decision tree.

In some embodiments, the ML predictive model is retrained with updated data and results from regulatory bodies as it becomes available, at set intervals, or the like.

Using the logical relationships, the hierarchal tree structure of the drug MOA relationships may be created (e.g., the hierarchical tree structure). The hierarchal tree structure may be created by interconnecting the direct relationships between respective MOAs (e.g., by modeling all associated relationships between the drug MOAs). The system may determine linear representation of the drug mechanisms of action hierarchies (e.g., by utilizing the first NLP model 148). The system may, for example, receive the hierarchical tree structure and break the tree structure down into a set of linear representations.

In certain embodiments, the determined linear representations are fed into a frequent pattern mining algorithm, e.g., the FP Growth algorithm, that utilizes a divide-and-conquer approach to provide insights about the frequency of occurrences of keywords and their relationships.

MOA clusters may be determined of the linear representations determined at step. For example, in some embodiments, the linear representations and the association rule data may be fed as input to unsupervised Gaussian Mixture Model (GMM), K-Means clustering, and/or hierarchical clustering algorithms (e.g., the ML clustering model 150). In some embodiments, the raw text fields are be transformed into a document-term matrix, e.g., based on a scikit-learn TfidfVectorizer.

The machine learning model may receive training data which may be based on previous clinical trials for pharmaceutical drugs. In some embodiments, the training data is partially labeled prior to receiving the data.

In one aspect, a computer-implemented method for generating machine learning training data may include: obtaining mechanism of action (MOA) data that is indicative of a hierarchical tree structure of relationships between the MOA data; generating linear representations of branches of the hierarchical tree structure; determining association rules for the MOA data by applying one or more frequent pattern mining algorithm to the linear representations; and determining, as at least a portion of the generated machine learning training data, MOA clusters by applying a clustering model to the linear representations and the association rules.

In another aspect, a computer-implemented method for predicting whether a post-marketing requirement (PMR) will be imposed on a clinical trial may include: obtaining data associated with a clinical trial; obtaining mechanism of action (MOA) data that is indicative of a hierarchical tree structure of relationships between the MOA data; generating a linear representation of one or more branches of the hierarchical tree structure; and generating a prediction of whether a PMR will be imposed on the clinical trial, by applying a trained machine learning model to the data associated with the clinical trial and the linear representation of the one or more branches, the trained model having been trained based on clusters of linear representations of historical MOA data.

In a further aspect, a system for predicting whether a post-marketing requirement (PMR) will be imposed on a clinical trial may include: one or more processors; and a non-transitory computer-readable medium storing instructions that are executable by the one or more processors to perform operations. The operations may include: obtaining data associated with a clinical trial; obtaining mechanism of action (MOA) data that is indicative of a hierarchical tree structure of relationships between the MOA data; generating a linear representation of one or more branches of the hierarchical tree structure; and generating a prediction of whether a PMR will be imposed on the clinical trial, by applying a trained machine learning model to the data associated with the clinical trial and the linear representation of the one or more branches, the trained model having been trained based on clusters of linear representations of historical MOA data.

It is to be understood that both the foregoing general description and the following detailed description are exemplary and explanatory only and are not restrictive of the disclosed embodiments, as claimed.

### BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.
FIG. 1A depicts an exemplary architecture for analyzing clinical trial data, according to one or more embodiments.
FIG. 1B depicts an exemplary architecture for a data processing module, according to one or more embodiments.
FIG. 1C depicts an exemplary environment architecture for implementing feature engineering and a machine learning model, according to one or more embodiments.
FIG. 2 depicts an exemplary mechanism of action (MOA) hierarchical tree structure, according to one or more embodiments.
FIG. 3 depicts an exemplary method for determining MOA clusters as training data for a machine learning system, according to one or more embodiments.
FIG. 4 depicts an exemplary relationship graph of determined MOA clusters, according to one or more embodiments.
FIG. 5A depicts an exemplary method for training a machine learning model, according to one or more embodiments.
FIG. 5B depicts an exemplary method of using a machine learning model, according to one or more embodiments.
FIG. 6A depicts another exemplary method for training a machine learning model, according to one or more embodiments.
FIG. 6B depicts another exemplary method of using a machine learning model, according to one or more embodiments.
FIG. 7 depicts a simplified functional block diagram of a computer, according to one or more embodiments

### DETAILED DESCRIPTION OF EMBODIMENTS

According to certain aspects of the disclosure, methods and systems are disclosed for generating a machine learning model for predicting whether a PMR will be imposed on a clinical trial. As will be discussed in more detail below, in various embodiments, systems and methods are described for reducing complex hierarchical tree structures for effective predictive MOA clusters that may be received as training data by a machine learning model.

Reference to any particular activity is provided in this disclosure only for convenience and not intended to limit the disclosure. The disclosure may be understood with reference to the following description and the appended drawings, wherein like elements are referred to with the same reference numerals.

The terminology used below may be interpreted in its broadest reasonable manner, even though it is being used in conjunction with a detailed description of certain specific examples of the present disclosure. Indeed, certain terms may even be emphasized below; however, any terminology intended to be interpreted in any restricted manner will be overtly and specifically defined as such in this Detailed Description section. Both the foregoing general description and the following detailed description are exemplary and explanatory only and are not restrictive of the features, as claimed.

In this disclosure, the term "based on" means "based at least in part on." The singular forms "a," "an," and "the" include plural referents unless the context dictates otherwise. The term "exemplary" is used in the sense of "example" rather than "ideal." The terms "comprises," "comprising," "includes," "including," or other variations thereof, are intended to cover a non-exclusive inclusion such that a process, method, or product that comprises a list of elements does not necessarily include only those elements, but may include other elements not expressly listed or inherent to such a process, method, article, or apparatus. The term "or" is used disjunctively, such that "at least one of A or B" includes, (A), (B), (A and A), (A and B), etc. Relative terms, such as, "substantially" and "generally," are used to indicate a possible variation of ±10% of a stated or understood value.

It will also be understood that, although the terms first, second, third, etc. are, in some instances, used herein to describe various elements, these elements should not be limited by these terms. These terms are only used to distinguish one element from another. For example, a first contact could be termed a second contact, and, similarly, a second contact could be termed a first contact, without departing from the scope of the various described embodiments. The first contact and the second contact are both contacts, but they are not the same contact.

As used herein, the term "if' is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Terms like "provider," "medical provider," or the like generally encompass an entity, person, or organization that may seek information, resolution of an issue, or engage in any other type of interaction with a patient, e.g., to provide medical care, medical intervention or advice, or the like. Terms like "patient," "client," "participant," "study participant" or the like generally encompass any person or entity who is obtaining information, seeking resolution of an issue, or engaging in any other type of interaction with a medical provider, e.g., to receive such care, information, or the like. Terms like "user" generally encompass any person or entity that may obtain information, resolution of an issue, purchase of a product, or engage in any other type of interaction with a provider or patient, whereby the user may be the medical provider or the patient as the case may be. For example, an individual that collects data on themselves may be both a patient and a user. A user may also refer to a patient that is being assisted by a provider. Further, a user may refer to a provider entering data on behalf of a patient.

As used herein, the term "post-marketing requirement (PMR)" is understood to encompass, for example, post-marketing requirements (PMRs), post-marketing commitments (PMCs), or post-authorization studies (PAS). Similarly, the terms "imposed," "imposing," "mandated," "mandating," "required," "requiring," or other variations thereof, when used in relation to a post-marketing requirement (PMR), are, optionally, construed to encompass PMRs that may not be strictly mandated but would be beneficial for obtaining regulatory approval, depending on the context.

International pharmaceutical regulators, such as the U.S. Food & Drug Administration (FDA) and the European Medicines Agency (EMA), may have guidelines and processes for deciding on when and how to impose additional research requirements after the marketing approval of a drug. The system described herein may implement machine learning techniques to predict whether a post-marketing requirement (PMR) may be imposed on a particular drug study, whereby the PMR may be mandated by a regulatory authority to approve a pharmaceutical drug.

It may be valuable for a drug manufacture to proactively plan for PMR during the approval process of a particular drug. If a drug manufacture can anticipate the demands of regulatory authorities, this may allow for the drug manufacture to define a mitigation strategy early in the drug development cycle or even avert the need for a post-marketing follow-up. For example, if the pharmaceutical sponsor can predict that a PMR will be called for and/or have some insight into as to why, the pharmaceutical sponsor can alter earlier phases of the clinical trial process to capture additional data and, for example, reduce and/or completely avert the PMR. Accurate and early foresight of PMRs can be critical to the overall success of a new product. However, it may be a time-consuming process to evaluate a pharmaceutical asset against the detailed guidelines provided by the FDA or EMA, and difficult to arrive with strong certainty in advance that a PMR might arise. Therefore, it may be valuable to have a system or method that can quickly and accurately predict whether a PMR will likely be required for a particular pharmaceutical asset.

One or more embodiments may include an operation to predict, for a particular pharmaceutical asset, whether a PMR will be required by a regulator. In some embodiments, the system includes a data processing module, a machine learning model, and an interactive user interface. The data processing module may receive real word data (RWD) and regulatory data and execute software to process the data. In certain embodiments, the data has feature engineering applied to process the data prior to input within the machine learning model. In some embodiments, the machine learning model is configured to output a prediction, e.g., a percentage chance that a particular pharmaceutical asset will require PMR, and the interactive user interface outputs the prediction to one or more users.

One or more embodiments may include an operation to apply feature engineering techniques to the RWD to further process the data prior to input in a machine learning system. For example, in certain embodiments, the RWD includes a drug's mechanism(s) of action (MOA). The MOA may refer to the specific biochemical process or processes by which a drug produces its pharmacological effect. Some embodiments of the system described herein determine drug MOA hierarchies and further reduce the MOA hierarchical tree structures for generating effective predictive MOA clusters by utilizing ensemble techniques, as will be described in greater detail below.

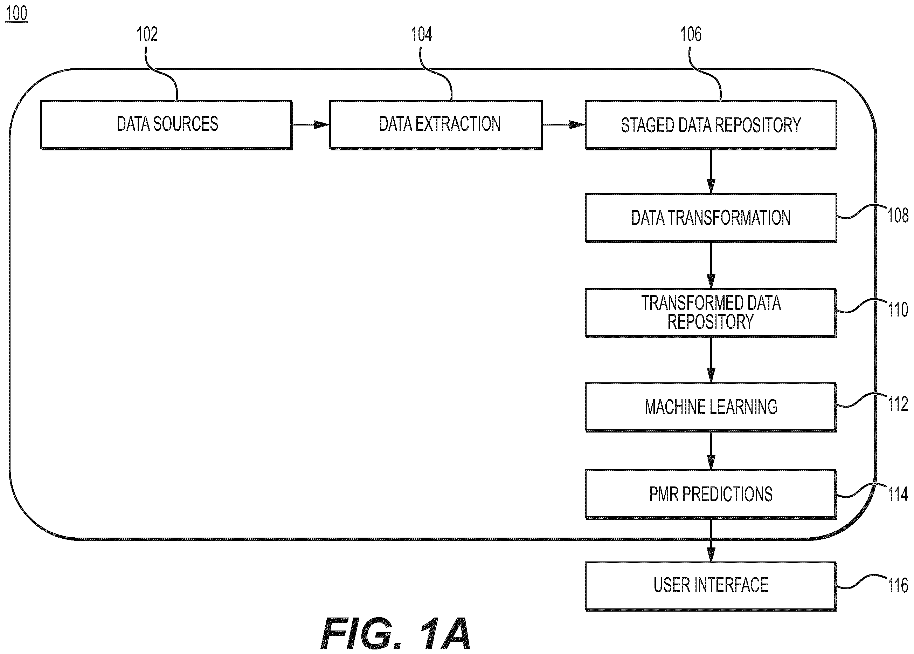

FIG. 1A depicts an exemplary architecture for analyzing clinical trial data, according to one or more embodiments. The exemplary architecture includes a system 100 that may be interconnected and accessed by a user via a network, such as the Internet or a cloud service provider, through one or more computers, servers, and/or handheld mobile devices.

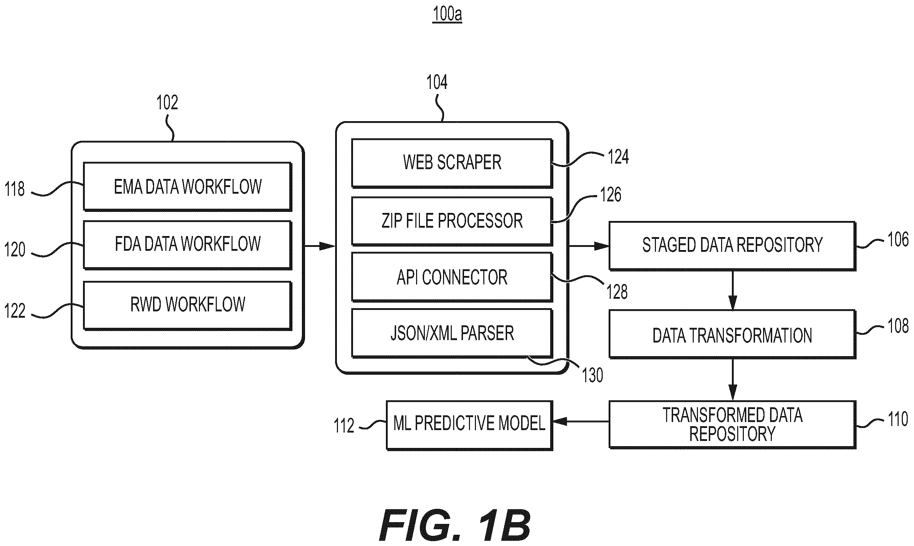

In some embodiments, the system 100 is configured to receive data from one or more data sources 102. The one or more data sources 102 may, for example, output RWD along with regulatory data as will be further discussed in FIG. 1B below. The RWD may include data of completed, in progress, and planned clinical trials. The regulatory data may include guidelines provided by regulators to analyze a study and/or determine whether to apply PMRs.

In certain embodiments, the system 100 may further be configured to perform data extraction through a data extraction model 104 from the one or more data sources 102. The data extraction model 104 may include web scrapers, zip file processors, an application programming interface (API) connector, a JavaScript Object Notation (JSON)/Extensible Markup Language (XML) parser, or any other suitable automatic and/or manual procedure, as described in greater detail in FIG. 1B. In some embodiments, the extracted data is stored in a staged data repository 106. The staged data repository 106 may be used to store the extracted RWD and regulatory data.

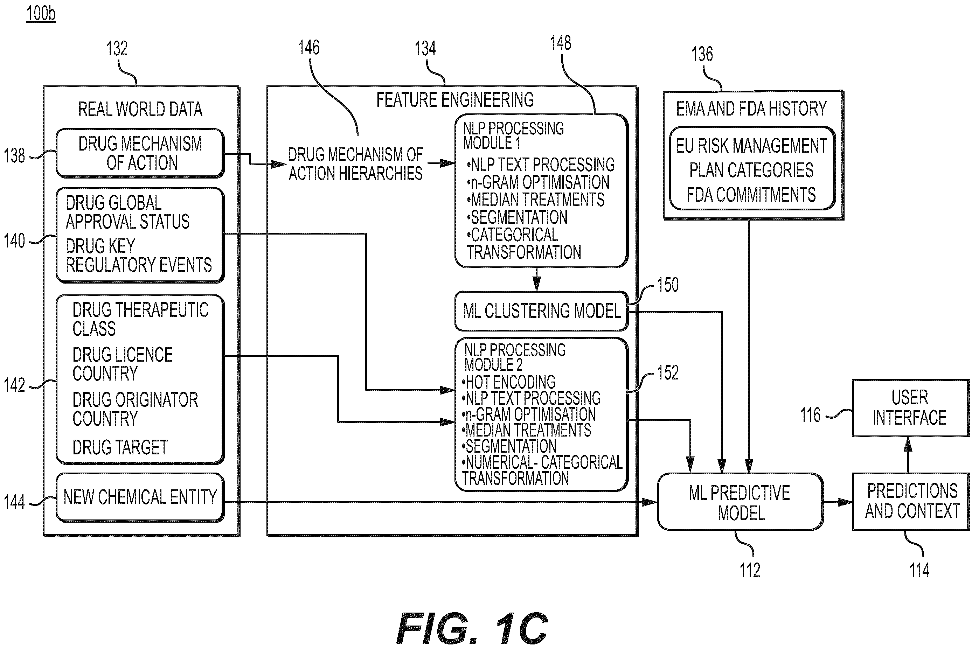

In some embodiments, the extracted data is sent to a data transformation model 108. In certain embodiments, the data transformation model 108 processes the data prior to inputting the data in the machine learning (ML) predictive model 112. The data transformation model 108 is described in greater detail below in FIG. 1C.

In some embodiments, the transformed data from the data transformation model 108 is stored in a transformed data repository 110.

For some embodiments, the system 100 includes a ML predictive model 112. The ML predictive model 112 may, for example, receive processed data from the transformed data repository 110. In certain embodiments, the ML predictive model 112 implements techniques such as gradient-boosting decision tree methodology, incorporating training data such as the data engineered in the other processing steps to identify factors associated with the occurrence of PMRs. Once trained, the ML predictive model 112 may provide as output, a PMR prediction 114 for any drug query made by an end user of the system. Each prediction 114 may be expressed as a probability score, which may be determined by the ML predictive model 112. The PMR prediction 114 may then, for example, be output to a user interface 116.

In some embodiments, the user interface 116 enables one or more users to access the system 100 to perform searches of a pharmaceutical drug. For example, a user may perform a search of the system 100 based on an originator license, company, drug, drug family, and/or drug MOA. Further, in certain embodiments, the user interface 116 is configured to display searched-for information, related drug MOA, and probability of an FDA or EMA post-marketing requirement (PMR) for the drug(s) in question. The end user may search the user interface 116 further based on MOA to find all corresponding clinical trials, and the display may provide details on any historical post-marketing studies and the types of studies mandated.

FIG. 1B depicts an exemplary architecture 100a for a one or more data sources 102 and data extraction models 104, according to one or more embodiments.

The one or more data sources 102 may include, for example, an EMA data workflow 118, a FDA data workflow 120, and/or a RWD workflow 122. Other or alternate data sources may be included in various embodiments. A data workflow orchestrator, which may be directed by a scheduler, may execute software to process the EMA data workflow 118, the FDA data workflow 120, and/or the RWD workflow 122. The data extraction models 104 may include a web scraper 124, a zip file processor 126, an API connector 128, and/or a JSON/XM1 Parser 130. Other data extraction models may be included in various embodiments. The data extraction models 104 may be configured to extract data from the one or more data sources 102.

The EMA may have electronic repositories of data collected from real-world data sources and studies that may include EMA post-authorization studies (PAS), and that are available publicly through the HMA-EMA Catalogues of real-world data sources and studies. The EMA data workflow 118 may, for example, receive data from this source. The web scraper 124 may, for example, scrape the data from the EMA website.

The FDA data workflow 120 may include data received from a downloadable spreadsheet detailing FDA decisions on PMRs on its public website. The FDA data workflow 120 may be executed by using the zip file processor 126.

The RWD workflow 122 may include RWD. Exemplary RWD that may be received includes, but is not limited to: drug MOAs, drug global approval status, drug key regulatory events, drug therapeutic class, drug license country, drug originator country, drug target, and/or the chemical entity of the particular drug. The public RWD on drugs and their MOA may be extracted and/or processed through the data extraction models 104, which may utilize REST API connectors 128 combined with JSON and XML parsers 130.

The extracted data may be staged in a staging database (i.e., the staged data repository 106), from where the data is joined using relevant keys, transformed and saved into a transformed data repository for consumption by the ML model as described in FIG. 1C below.

The system 100 may, for example, perform scheduled data capture (e.g., from the one or more data sources 102) at set time intervals. For example, data may be extracted from the EMA data workflow 118 and from the FDA data workflow 120 at set interval such as once per day. In another example, data capture may be automatic, e.g., performed in response to detection of new data being available. In various embodiments, data capture may be performed in response to a user instruction, manually, and/or via any other suitable technique.

FIG. 1C depicts an exemplary environment architecture for implementing a feature engineering model 134 and a ML predictive model 112, according to one or more embodiments.

In some embodiments, the real world data 132 of FIG. 1C includes data extracted from the RWD workflow 122. The EMA and FDA history data 136 may, for example, include data extracted from the EMA data workflow 118 and the FDA data workflow 120. The feature engineering model 134 may, for example, be applied by the data transformation model 108. The output of the feature engineering model 134, along with RWD (e.g., new chemical entity 144) and the EMA and FDA history data 136 may be fed to the ML predictive model 112 for analysis.

In certain embodiments, the feature engineering model 134 receives real world data 132 for processing. For example, the feature engineering model 134 may receive drug MOA data 138, drug global approval status and drug key regulatory event data 140, drug therapeutic class, drug license country, drug originator country, and drug target data 142.

For some embodiments, the feature engineering model 134 receives drug MOA data 138. This may include some or all historical drug MOAs, their respective logical relationships, as well as MOAs related to a particular new pharmaceutical drug. The particular drug MOA relationships may include one or more parent/child relationships. Received drug MOAs may have associations that can be modeled using feature engineering. For example, in one embodiment, there may be 3,800 or more distinct drug MOAs with 9,800 or more associated synonyms, with a possible 782 or more parents, 185 or more grandparents, 34 or more great-grand-parents and 3 or more great-great-grand parents, etc. It should be understood that, as more MOAs are discovered and/or understood, MOA data 138 may be updated over time.

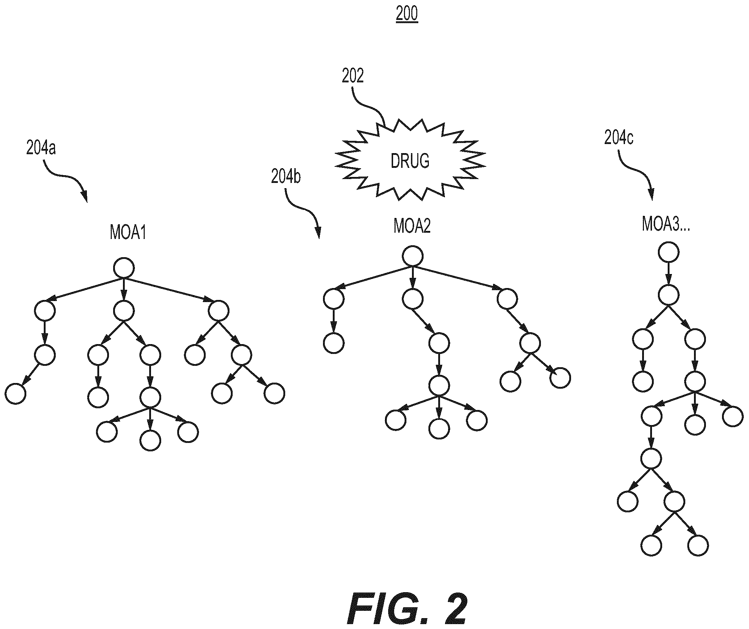

The feature engineering model 134 may, for example, model the MOA data 138 as drug MOA hierarchies 146 as represented in an example illustrated in FIG. 2. FIG. 2 depicts an illustrative representation of an exemplary mechanism of action (MOA) hierarchical tree structure 200 for an example drug, according to one or more embodiments. The hierarchical tree structure 200 may depict a multi-level tree structure with interconnected synonym nodes of MOAs. Some embodiments of the hierarchical tree structure 200 include the three great-great-grand parents 204a, 204b, 204c and all respective relationships of the drug MOAs. A new clinical drug 202 may, for example, be associated with one or more MOAs of the hierarchical tree structure 200.

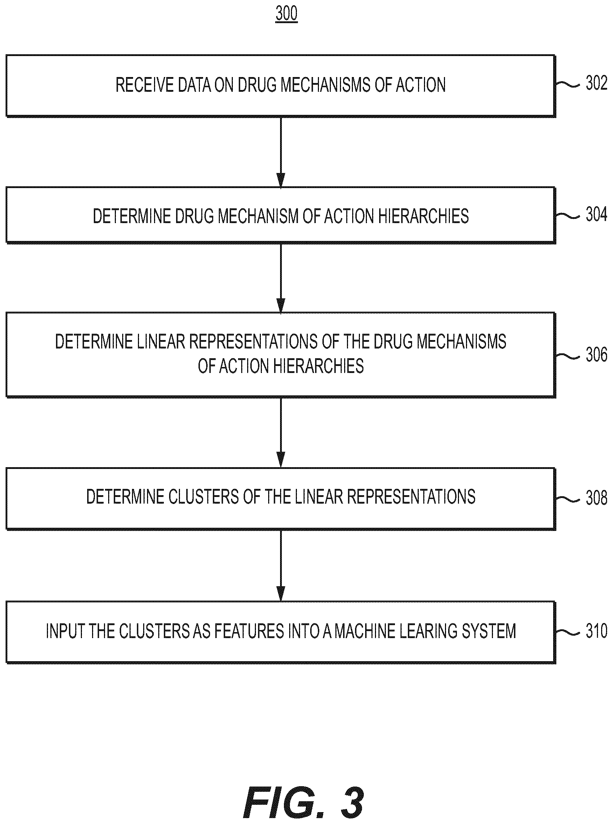

As will be described in greater detail in FIG. 3, in some embodiments, the first Natural Language Processing (NLP) model 148 is configured to reduce the drug MOA hierarchies 146 to linear representations. The first NLP model 148 may utilize one or more exemplary algorithms to reduce the MOA hierarchical tree structures to linear representations. For example, in certain embodiments, the first NLP model 148 utilize algorithms including, but not limited to: tokenization, vectorization, max and min n-gram limit determinations, word clouds, median treatment, segmentation, text classification, and/or categorical transformation. In some embodiments, the linear representations are then be fed into a frequent pattern mining algorithm, e.g., by the Frequent Pattern (FP) Growth algorithm, in order to extract association rules. As used herein, association rules generally encompass identified relationships between items in a given dataset representing patterns where items in a dataset frequently appear together, indicating that the presence of certain items (antecedents) likely predicts the presence of others (consequents). In some embodiments, association rules may be evaluated, e.g., based on a high confidence such as a factor greater than 50%, and where a consequent is set to pre-identified PMRs.

The outputs of the first NLP model 148, e.g., the linear representations and the association rule data, may, for example, be saved and/or fed into a machine learning clustering model 150. In some embodiments, the machine learning clustering model 150 utilize machine learning algorithms such as clustering to categorize the MOAs into a set of distinct mechanism groups, e.g., based on silhouette scores. In certain embodiments, raw text fields are transformed into a document-term matrix, e.g., based on a scikit-learn TfidfVectorizer. In some embodiments, the data are reduced to a binary representation in which frequency of the n-grams is not the deterministic factor. In alternate embodiments, a word analyzer is be utilized, e.g., instead of a character analyzer, to speed up the process. The MOA clusters clustered groups may then be output, e.g., in a numerical representation, to the ML predictive model 112 for further analysis. An experimental result of the foregoing procedure resulted in clusters having strong relationships with therapeutic indicators.

Such a procedure may enable effective traveling across every node of the complex MOA forest to identify probable keywords or relational keywords associated with historical PMRs without increasing the time and cost required for the node travel.

In some embodiments, the feature engineering model 134 also has a second NLP model 152. The second NLP model 152 may, for example, receive RWD of drug regulatory events, drug therapeutic classes, drug targets, and drug licensing and origin from real world data 132. The second NLP model 152 may be configured to embed text variables as numeric vectors. For example, the second NLP model 152 may apply techniques including, but not limited to: hot encoding, text processing, n-Gram optimization, median treatments, segmentation, numerical-categorical transformation. The outputs of certain embodiments of the second NLP model 152 are fed to the ML predictive model 112, e.g., in combination with the other data discussed above, such as the MOA data, numerical representation of clusters, etc.

In some embodiments, the ML predictive model 112 is configured to receive a new chemical entity 144, along with the output from the ML clustering model 150, the output from the second NLP module 152, and the EMA and FDA history data 136. The ML predictive model 112 may be configured to utilize a gradient-boosting decision tree methodology to use the training data generated in the other processing steps to identify factors associated with the occurrence of PMRs. Once trained, the ML predictive model 112 may provide a PMR prediction 114 for any drug query made by an end user of the system, as outputted in the user interface 116. Each prediction 114 may be expressed as a probability score, which may be determined by the model's decision tree.

In some embodiments, the ML predictive model 112 is retrained with updated data and results from regulatory bodies as it becomes available, at set intervals, or the like. For example, every week or every month, the system 100 may retrain the ML predictive model 112. Retraining the ML predictive model 112 may include incorporating PMR decisions for pharmaceutical trials where decisions were rendered since the last training was performed.

In some embodiments, the user interface 116 enables a user to interactively search for information related to a pharmaceutical asset. For example, a user can perform a search of the system based on an originator license, company, drug, drug family, and/or drug MOA. Further, the user interface 116 may be configured to display searched-for information, related drug MOA, and probability of an FDA or EMA post-marketing requirement (PMR) for the drug(s) in question. The end user may search the user interface 116 further based on MOA to find all corresponding clinical trials, and the display may provide details on any historical post-marketing studies and the types of studies mandated.

In its guidance for industry on post-marketing studies and clinical trials, the FDA has noted that a risk assessment during the approval process can lead to a request for a PMR to gather more information about the risk to better inform product labelling. Examples of PMRs are: observational pharmacoepidemiologic studies, meta-analyses of previously captured safety endpoint data, new clinical trials to capture safety endpoint data based on the serious risk identified by the FDA, safety studies in animals, or in vitro laboratory safety studies. The EMA may have similar guidance on good pharmacovigilance practice and impose corresponding PMRs, such as: observational pharmacoepidemiologic studies, additional risk-minimization measures, post-authorization safety studies (PASS), or post-authorization efficacy studies (PAES).

In some embodiments, the user interface 116 is configured to output PMR predictions 114 with more specific details as to which type of PMR is likely. For example, in various embodiments, the ML model may be trained to generate a separate prediction for each type of PMR, or predict a type of PMR given a generated percentage of a PMR and, e.g., the other data input into the ML model.

FIG. 3 depicts an exemplary method 300 for determining clusters as training data for a machine learning system (e.g., for the ML predictive model 112), according to one or more embodiments.

At step 302, the system described herein (e.g., the feature engineering model 134) may receive the drug MOAs (drug MOA data 138), the drug MOAs respective logical relationships, and/or particular MOA's associated with a new pharmacological drug.

At step 304, using the logical relationships, the hierarchal tree structure of the drug MOA relationships may be created (e.g., the hierarchical tree structure 200). The hierarchal tree structure may be created by interconnecting the direct relationships between respective MOAs (e.g., by modeling all associated relationships between the drug MOAs).

At step 306, the system may determine linear representation of the drug mechanisms of action hierarchies (e.g., by utilizing the first NLP model 148). The system may, for example, receive the hierarchical tree structure from step 304 and break the tree structure down into a set of linear representations. The linear representations may be determined by applying one or more of, a tokenization algorithm, vectorization, a max and min n-gram limit determination, word clouds, median treatments, segmentation, text classification, and categorical transformation.

In certain embodiments, the determined linear representations are fed into a frequent pattern mining algorithm, e.g., the FP Growth algorithm, that utilizes a divide-and-conquer approach to provide insights about the frequency of occurrences of keywords and their relationships. In some embodiments, association rules are extracted based on a high confidence factor greater than 50%, where the consequent is set to pre-identified PMRs.

At step 308, MOA clusters may be determined of the linear representations determined at step 306. For example, in some embodiments, the linear representations and the association rule data may be fed as input to unsupervised Gaussian Mixture Model (GMM), K-Means clustering, and/or hierarchical clustering algorithms (e.g., the ML clustering model 150). In some embodiments, the raw text fields are be transformed into a document-term matrix, e.g., based on a scikit-learn TfidfVectorizer. The data may be reduced to binary representation, in which the frequency of the n-grams may not be the deterministic factor. A word analyzer may be utilized instead of a character analyzer to speed up the process. A NLTK word tokenizer may be employed, which in turn utilizes scikit-learn vectorizer.

At step 308, a plurality, e.g., twelve to fifteen, clusters of linear representations may be determined.

At step 310, the determined MOA clusters may be fed to a machine learning system (e.g., to the ML predictive model 112), along with other data such as RWD or regulatory data. The determined MOA clusters may assist in the machine learning system what factors (e.g., what drug MOAs) associate with the occurrence of PMRs.

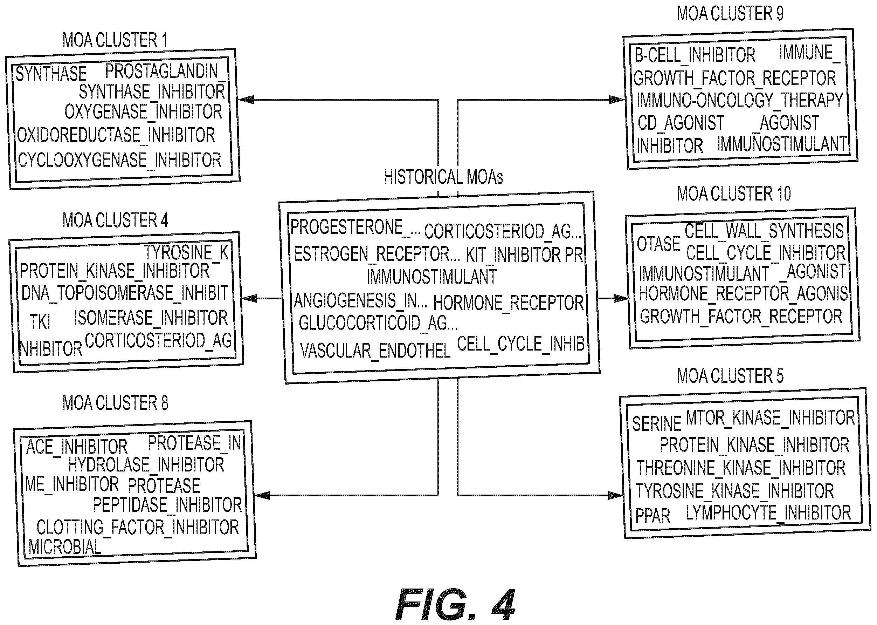

FIG. 4 depicts an exemplary relationship graph of determined MOA clusters 400, according to one or more embodiments. In some embodiments, the determined MOA clusters 400 are determined by the ML clustering model 150 and input as training data the ML predictive model 112 of FIG. 1C.

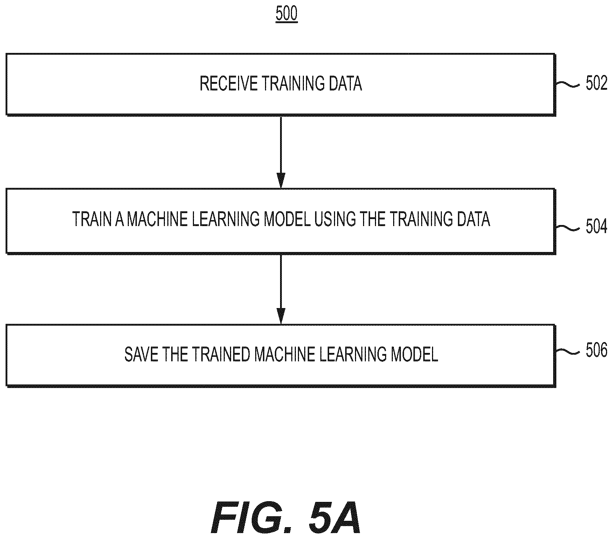

FIG. 5A depicts an exemplary method 500 for training a machine learning model (i.e., the ML predictive model 112 of FIG. 1A), according to one or more embodiments.

At step 502, the machine learning model may receive training data. The training data may, for example, be based on previous clinical trials for pharmaceutical drugs. The training data of various embodiments include RWD data such as drug MOAs, drug global approval status, drug key regulatory events, drug therapeutic class, drug license country, drug originator country, drug target, and the chemical entity of the particular drug. The training data of certain embodiments further include whether and what type of PMR was assigned to the particular clinical trial.

In some embodiments, the training data is partially labeled (e.g., the PMR result may be labeled and included) prior to receiving the data. In other embodiments, the system also extracts the PMR result from the one or more data sources of previously analyzed drugs and utilize this data for training.

At step 504, the machine learning system may be trained on the data received at step 502. In some embodiments, the received RWD data has feature engineering applied and input into the machine learning system. In certain embodiments, the machine learning system implements a gradient-boosting decision tree methodology, and uses the training data generated in the processing steps to identify RWD factors associated with the occurrence of PMRs. In certain embodiments, the machine learning system may be trained on historical drug cases, where the decision of whether to mandate PMR was previously determined,

At step 506, the trained machine learning model may be saved to storage for future use and training.

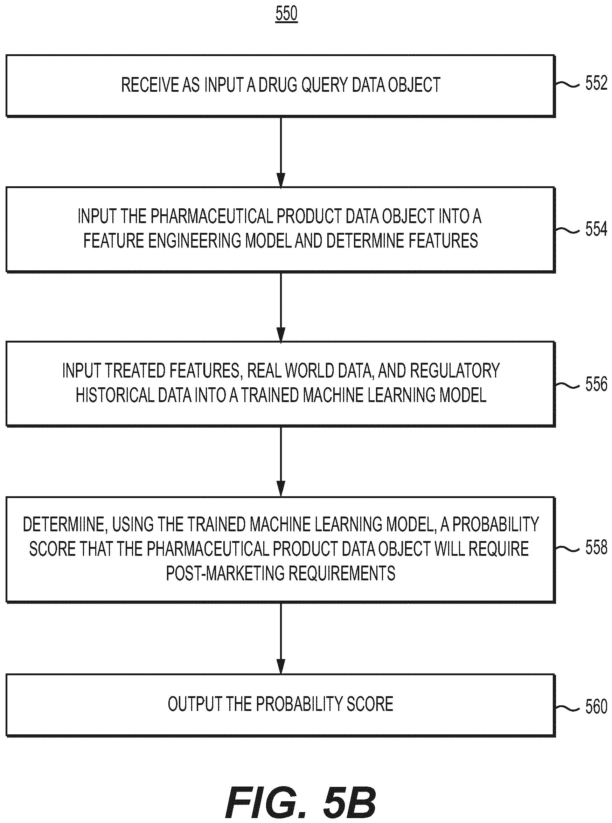

FIG. 5B depicts an exemplary method 550 of using a machine learning model (i.e., the ML predictive model 112 of FIG. 1A), according to one or more embodiments.

At step 552, the system may receive as input a new drug query data object. In some embodiments, this data relates to a new pharmaceutical drug/asset. In some embodiments, the new drug query data object corresponds to a drug that is about to, or is in the process, of receiving regulatory approval. Further, the RWD of the new drug query data object may be received. For example, in some embodiments, the RWD may include the drug MOAs, drug global approval status, drug key regulatory events, drug therapeutic class, drug license country, drug originator country, drug target, and the chemical entity of the particular drug.

At step 554, the RWD may, for example, be input into a feature engineering model (e.g., the feature engineering model 134). In some embodiments, the feature engineering model determines a linear representation of the drug MOA and identifies what MOA clusters are associated with the drug MOA utilizing the techniques discussed in method 300, FIG. 3.

Further, at step 554, RWD data such as the drug global approval status, drug key regulatory events, drug therapeutic class, drug license country, drug originator country, drug target may be input into a NLP model (e.g., the second NLP model 152). In some embodiments, the NLP processing module processes the RWD into text numeric vectors to be input to the ML predictive model.

At 556, the determined training data from step 554 (e.g., the output of ML clustering model 150 and the output of NLP processing module 2 152), RWD of the drug-query-data-object (e.g., the new chemical entity 144), and the regulatory historical data (e.g., the EMA and FDA history data 136) may be input into a trained ML prediction model. In some embodiments, this is the ML predictive model 112.

At step 558, the trained ML prediction model may, for example, determine a probability score that the drug query data object will require PMR.

At step 560, the PMR prediction and probability score may be saved and/or output (e.g., via the user interface 116).

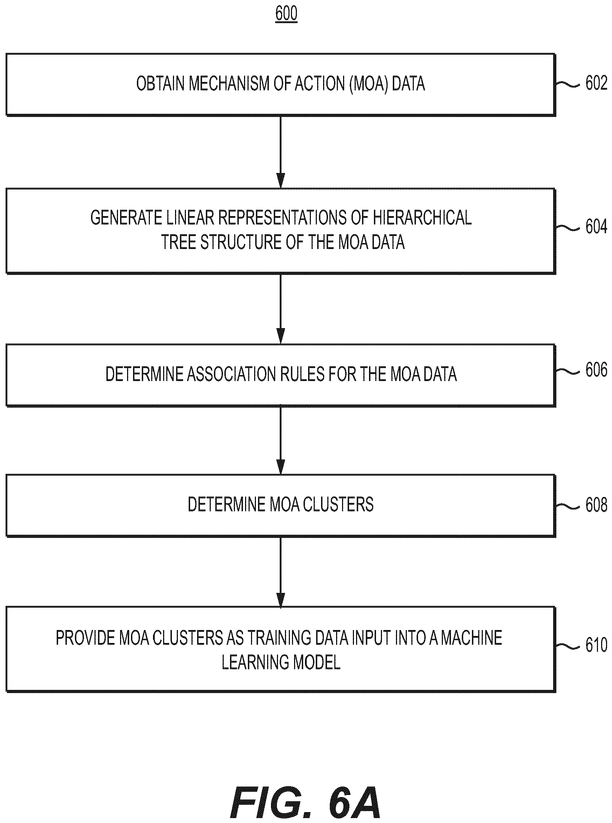

FIG. 6A depicts another exemplary method 600 for training a machine learning model (i.e., the ML predictive model 112 of FIG. 1A), according to one or more embodiments. At step 602, mechanism of action (MOA) data is obtained. The MOA data may be indicative of (e.g., may include or be usable to generate) a hierarchical tree structure of relationships between the MOA data. In some embodiments, the hierarchical tree structure is determined by, for example, extracting a plurality of nodes from the MOA data, and generating the hierarchical tree structure based on the extracted nodes.

At step 604, linear representations of branches of the hierarchical tree structure are generated. In some embodiments, generating linear representations of branches of the hierarchical tree structure includes applying one or more techniques selected from the group consisting of: tokenization, vectorization, max and min n-gram limit determination, word clouds, median treatments, segmentation, text classification, and categorical transformation.

At step 606, association rules for the MOA data are determined. The association rules may be generated by applying one or more frequent pattern mining algorithm to the linear representations. In some embodiments, the generation of association rules includes applying a Frequent Pattern (FP) Growth algorithm.

At step 608, MOA clusters are determined. The MOA clusters may be generated by applying a clustering model to the linear representations and the association rules, and may be used as training data input into a machine learning model. In certain embodiments, the generation of MOA clusters includes applying a clustering model selected from the group consisting of: a Gaussian Mixture Model (GMM), K-Means Clustering, and hierarchical clustering. In some embodiments, the MOA clusters are transformed into numerical representations. The machine learning training data of certain embodiments further includes a labeled dataset, indicating, for example, whether a post-marketing requirement (PMR) was imposed on a previous clinical trial.

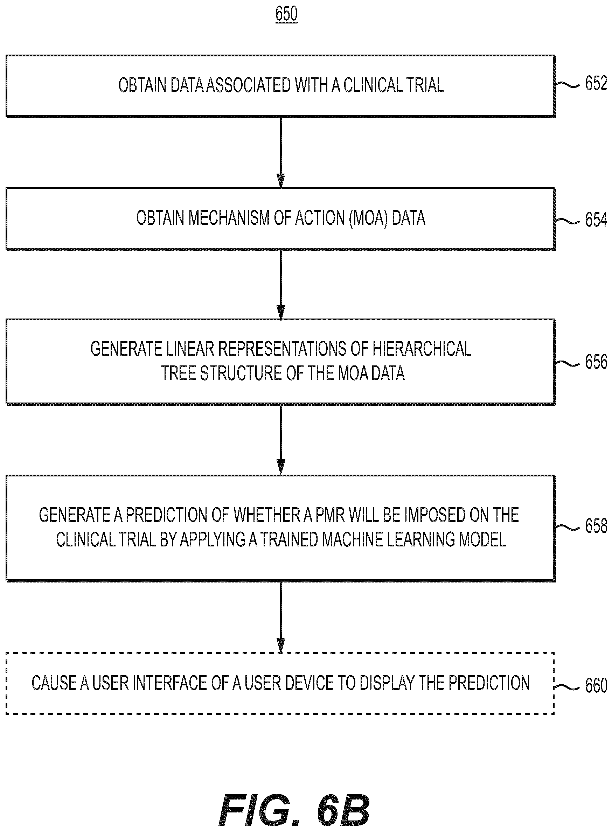

FIG. 6B depicts another exemplary method 650 of using a machine learning model (i.e., the ML predictive model 112 of FIG. 1A), according to one or more embodiments.

At step 652, data associated with a clinical trial is obtained (e.g., the new chemical entity 144). In some embodiments, the data associated with a clinical trial includes one or more of: global approval status, key regulatory events, therapeutic class, license country, originator country, and target.

At step 654 mechanism of action (MOA) data is obtained. The MOA data may be indicative of (e.g., may include or be usable to generate) a hierarchical tree structure of relationships between the MOA data. In some embodiments, the hierarchical tree structure is determined by, for example, extracting a plurality of nodes from the MOA data, and generating the hierarchical tree structure based on the extracted nodes.

At step 656, linear representations of branches of the hierarchical tree structure may be generated. In some embodiments, generating linear representations of branches of the hierarchical tree structure includes applying one or more techniques selected from the group consisting of: tokenization, vectorization, max and min n-gram limit determination, word clouds, median treatments, segmentation, text classification, and categorical transformation.

At step 658, a prediction of whether a PMR will be imposed on the clinical trial is generated by applying a trained machine learning model. The prediction of whether a PMR will be imposed on the clinical trial may be generated by applying the trained machine learning model to the data associated with the clinical trial and the linear representation of the one or more branches. The trained machine learning model may also include a gradient-boosting decision tree model. In some embodiments, the trained machine learning model is trained based on clusters of linear representations of historical MOA data. The trained machine learning model may, in certain embodiments, be further trained using regulatory data from one or more of the Food and Drug Administration (FDA) or the European Medicines Agency (EMA) (e.g., the EMA and FDA history data 136). The prediction can further include, as in some embodiments, an indication of a specific type of PMR likely to be imposed.

The trained machine learning model may, in some embodiments, be periodically retrained with updated data. The data used to retrain the machine learning model may include MOA data, linear representations of MOA data, clusters of linear representations of MOA, data associated with a clinical trial, and regulatory data.

In some embodiments, at a step 660, the prediction is displayed on a user interface of a user device (e.g., via the user interface 116). In certain embodiments, the user interface may also display details of historical post-marketing studies and types of studies mandated for drugs associated with a drug query.

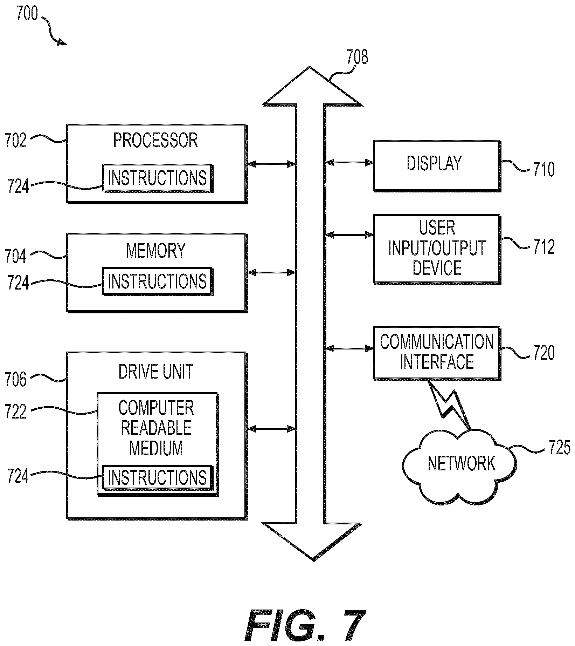

FIG. 7 depicts a simplified functional block diagram of a computer, according to one or more embodiments. The computer 700 of FIG. 7 may be utilized to implement the system 100 of FIG. 1A or to access the system 100 through a user interface 116. One or more of a processor 702, a memory 704, a drive unit 706, an internal communication bus 708, a display 710, a user input/output ports 712, a communication interface 720, a computer readable medium 722, instructions 724, and a network 725 may communicate by any suitable means. For example, computer 700 may be configured as the one or more sensors, patient communication device, and/or another system according to exemplary embodiments of this disclosure. In various embodiments, any of the systems herein may be a computer 700 including, for example, data communication interface 720 for packet data communication. Computer 700 also may include a central processing unit (CPU) 702, in the form of one or more processors, for executing program instructions. Computer 700 may include internal communication bus 708, and storage unit 706 (such as Read-Only Memory (ROM), Hard Disk Drive (HDD), Solid-State Drive (SSD), etc.) that may store data on computer readable medium 722, although computer 700 may receive programming and data via network communications. Computer 700 may also have memory 704 (such as Random-Access Memory (RAM)) storing instructions 724 for executing techniques presented herein, although instructions 724 may be stored temporarily or permanently within other modules of computer 700 (e.g., processor 702 and/or computer readable medium 722). Computer 700 also may include input and output ports 712 and/or display 710 to connect with input and output devices such as keyboards, mice, touchscreens, monitors, displays, etc. The various system functions may be implemented in a distributed fashion on a number of similar platforms, to distribute the processing load. Alternatively, the systems may be implemented by appropriate programming of one computer hardware platform.

Program aspects of the technology may be thought of as "products" or "articles of manufacture" typically in the form of executable code and/or associated data that is carried on or embodied in a type of machine-readable medium. "Storage" type media include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer of the mobile communication network into the computer platform of a server and/or from a server to the mobile device. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links, or the like, also may be considered as media bearing the software. As used herein, unless restricted to non-transitory, tangible "storage" media, terms such as computer or machine "readable medium" refer to any medium that participates in providing instructions to a processor for execution.

While the disclosed methods, devices, and systems are described with exemplary reference to transmitting data, it should be appreciated that the disclosed embodiments may be applicable to any environment, such as a desktop or laptop computer, an automobile entertainment system, a home entertainment system, medical equipment, etc. Also, the disclosed embodiments may be applicable to any type of Internet protocol.

It should be appreciated that in the above description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments may be used in any combination.

Thus, while certain embodiments have been described, those skilled in the art will recognize that other and further modifications may be made thereto without departing from the spirit of the invention, and it is intended to claim all such changes and modifications as falling within the scope of the invention. For example, functionality may be added or deleted from the block diagrams and operations may be interchanged among functional blocks. Steps may be added or deleted to methods described within the scope of the present invention.

The above disclosed subject matter is to be considered illustrative, and not restrictive, and the appended claims are intended to cover all such modifications, enhancements, and other implementations, which fall within the true spirit and scope of the present disclosure. Thus, to the maximum extent allowed by law, the scope of the present disclosure is to be determined by the broadest permissible interpretation of the following claims and their equivalents, and shall not be restricted or limited by the foregoing detailed description. While various implementations of the disclosure have been described, it will be apparent to those of ordinary skill in the art that many more implementations are possible within the scope of the disclosure. Accordingly, the disclosure is not to be restricted except in light of the attached claims and their equivalents.

## Claims

1. A computer-implemented method for generating machine-learning training data, the method being carried out by a system comprising one or more processors (702) and a non-transitory computer-readable medium (704, 722) storing instructions that are executable by the one or more processors to perform operations; and the method comprising:
obtaining (654) mechanism of action (MOA) data that is indicative of a hierarchical tree structure of relationships between the MOA data (138);
generating (656) linear representations of branches of the hierarchical tree structure;
determining (606) association rules for the MOA data by applying one or more frequent pattern mining algorithm to the linear representations; and
determining (308, 608), as at least a portion of the generated machine learning training data, MOA clusters (400) by applying a clustering model (150) to the linear representations and the association rules.

2. A computer-implemented method of claim 1, further comprising determining the hierarchical tree structure by:
extracting (104) a plurality of nodes from the MOA data (138); and
generating (602) the hierarchical tree structure based on the extracted nodes.

3. A computer-implemented method of claim 1 or claim 2, wherein generating (604) linear representations of branches of the hierarchical tree structure includes applying one or more techniques selected from the group consisting of: tokenization, vectorization, max and min n-gram limit determination, word clouds, median treatments, segmentation, text classification, and categorical transformation.

4. A computer-implemented method of any preceding claim, wherein determining (606) association rules for the linear representations comprises applying a Frequent Pattern (FP) Growth algorithm.

5. A computer-implemented method of any preceding claim, wherein determining (608) MOA clusters comprises applying a clustering model selected from the group consisting of: a Gaussian Mixture Model (GMM), K-Means Clustering, and hierarchical clustering.

6. A computer-implemented method of any preceding claim, further comprising transforming (608) the MOA clusters into numerical representations for use as input into a machine learning model.

7. A computer-implemented method of any preceding claim, wherein the machine learning training data further includes a labeled dataset indicating whether a post-marketing requirement (PMR) was imposed on a previous clinical trial.

8. A computer-implemented method as claimed in claim 7, wherein the method includes predicting (114) whether a post-marketing requirement (PMR) will be imposed on a clinical trial, the method comprising:
obtaining (654) data associated with a clinical trial, and said (MOA) data is associated with the clinical trial, and
generating (658) a prediction of whether a PMR will be imposed on the clinical trial by applying a trained machine learning model to the data associated with the clinical trial and the linear representation of the one or more branches, the trained machine learning model having been trained based on clusters of linear representations of historical MOA data according to said generated machine learning training data.

9. A computer-implemented method of claim 8, wherein the data associated with the clinical trial includes one or more of: global approval status, key regulatory events, therapeutic class, license country, originator country, and target; and wherein the trained machine learning model was further trained (504) using regulatory data from one or more of the Food and Drug Administration (FDA) or the European Medicines Agency (EMA).

10. A computer-implemented method of claim 8 or claim 9, wherein the trained machine learning model includes a gradient-boosting decision tree model.

11. A computer-implemented method of any of claims 8 to 10, wherein the prediction further includes an indication of a specific type of PMR likely to be imposed.

12. A system (700) for predicting whether a post-marketing requirement (PMR) will be imposed on a clinical trial, the system comprising:
one or more processors (702);
a user interface (116), and
a non-transitory computer-readable medium (704, 722) storing instructions that are executable by the one or more processors to perform operations, including:
obtaining data associated with a clinical trial;
obtaining (654) mechanism of action (MOA) data (138) associated with the clinical trial, the MOA data indicative of a hierarchical tree structure of relationships between the MOA data;
generating (656) a linear representation of one or more branches of the hierarchical tree structure;
generating a prediction (114) of whether a PMR will be imposed on the clinical trial,
by applying a trained machine learning model to the data associated with the clinical trial and the linear representation of the one or more branches, the trained machine learning model having been trained based on clusters of linear representations of historical MOA data, and
outputting the prediction at the user interface (116).

13. A system of claim 12, wherein the data associated with the clinical trial includes one or more of: global approval status, key regulatory events, therapeutic class, license country, originator country, and target.

14. A system of claim 12 or claim 13, wherein the one or more processors are configured to periodically retrain the trained machine learning model (112) with updated data, wherein the updated data includes one or more of: MOA data, linear representations of MOA data, clusters of linear representations of MOA, data associated with a clinical trial, and regulatory data.

15. A system of any of claims 12 to 14, further comprising:
an interactive user interface configured to receive a drug query from a user and to display a prediction of whether a PMR will be imposed on a clinical trial associated with the drug query; wherein the interactive user interface is further configured to display an indication of a specific type of PMR likely to be imposed; and
wherein the interactive user interface is further configured to display details of historical post-marketing studies and types of studies mandated for drugs associated with the drug query.
